# FASCICULE DE BREVET EUROPEEN

(11) **EP 2 381 931 B1**
(45) Date de publication et mention de la délivrance du brevet: **12.02.2020**
(21) Numéro de dépôt: 09805748.2
(22) Date de dépôt: 18.12.2009
(51) Int. Cl.: A61K 9/50, A61K 9/51

(54) **COMPOSITION COMPRENANT UN ACTIF DE FAIBLE SOLUBILITÉ AQUEUSE**
ZUSAMMENSETZUNG MIT EINEM WIRKSTOFF MIT GERINGER WASSERLÖSLICHKEIT
COMPOSITION INCLUDING AN ACTIVE AGENT WITH LOW WATER SOLUBILITY

(30) Priorité: 31.12.2008 FR 0859170; 31.12.2008 US 141834 P
(43) Date de publication de la demande: 02.11.2011
(73) Titulaire: Flamel Ireland Limited, Dublin 15 (IE)
(72) Inventeur: MEYRUEIX, Rémi, 69009 Lyon (FR); JORDA, Rafael, 33700 Merignac (FR); POULIQUEN, Gauthier, 69007 Lyon (FR); CHAN, You-Ping, 69360 Ternay (FR); BREYNE, Olivier, 69004 Lyon (FR)
(74) Mandataire: Nony
(86) Numéro de dépôt international: PCT/FR2009/052613
(87) Numéro de publication internationale: WO 2010/076519

(56) Documents cités:
- WO-A-2008/025425
- WO-A2-2010/012940
- FR-A- 2 786 098
- FR-A- 2 840 614
- FR-A- 2 891 459
- FR-A- 2 892 725

## Description

La présente invention vise à proposer des compositions, appropriées à la formulation de principe(s) actif(s), abrégé en « PA », notamment de faible solubilité aqueuse. Elle vise également à proposer un mode de formulation efficace pour, de manière générale, accroître la solubilisation aqueuse de tout principe actif.

Comme décrit ci-après, un grand nombre d'actifs, qu'ils soient thérapeutiques, prophylactiques ou cosmétiques, peuvent poser des problèmes en termes de formulation au regard d'une solubilité aqueuse estimée insuffisante.

En particulier, il s'avère très difficile de formuler des actifs faiblement hydrosolubles sous une forme compatible avec une administration par voie orale, voie particulièrement appréciée pour l'administration d'actifs, notamment au regard de son confort pour le patient et de sa compatibilité avec une grande variété de formulations.

Ainsi, les principes actifs de classes 2 ou 4 de la classification biopharmaceutique ont pour l'essentiel une biodisponibilité orale limitée par leur faible solubilité. En particulier, le paclitaxel, taxoïde naturel, largement utilisé pour le traitement des tumeurs est représentatif de ces actifs peu hydrosolubles. La très faible solubilité aqueuse de ce composé, inférieure à 1 µg/ml, rend difficile sa formulation.

Dans ce contexte, le développement d'additifs permettant d'accroître la solubilité aqueuse de principes actifs est d'un intérêt considérable.

Idéalement un additif de solubilisation doit présenter plusieurs caractéristiques essentielles.

Tout d'abord, il doit, pour des raisons évidentes, posséder un pouvoir de solubilisation élevé. Ainsi, il est nécessaire que le polymère solubilise une quantité suffisamment importante de PA. Ceci présente deux avantages. Cette aptitude permet de minimiser la quantité d'additif, ce qui peut être crucial pour la tolérance dans le cas d'une forme parentérale. Par ailleurs, une solubilité élevée permet de rendre la dose unitaire aisément administrable chez le patient, que ce soit par voie orale ou parentérale.

Par ailleurs, l'additif de solubilisation en tant que tel, de même que le protocole de formulation retenu pour l'associer au principe actif considéré, ne doivent pas dégrader l'actif. Ainsi, il est déterminant que les différentes étapes de la formulation de l'actif et de l'additif de solubilisation ne conduisent pas à une dégradation partielle de l'actif. Une dégradation potentielle pourrait se produire par exemple lors d'une élévation de température, par l'utilisation de tensioactifs, la mise au contact de l'actif avec un solvant organique, ou un cisaillement élevé. Or, certains actifs, en particulier des peptides et des protéines sont particulièrement sensibles à ces modes de dégradation. Il est donc particulièrement important que l'additif de solubilisation puisse être mis en œuvre dans un procédé aqueux ne nécessitant pas de température excessive, de tensioactif, ni de solvant organique.

D'autre part, il est avantageux que la formulation de l'actif avec un additif de solubilisation possède une faible viscosité. Ainsi, pour un principe actif destiné à une de administration parentérale, la viscosité de la suspension contenant l'actif et le solubilisant doit être suffisamment basse pour permettre une injection aisée au travers d'une aiguille de faible diamètre, par exemple une aiguille de jauge 27 à 31. En fait, même dans le cas d'une administration orale d'un PA contenu dans un comprimé, une basse viscosité de la suspension solubilisant l'actif reste un avantage décisif pour les étapes de fabrication de microparticules, de comprimés ou de toute autre forme pharmaceutique connue de l'homme de l'art. Cette exigence de faible viscosité est particulièrement contraignante car elle limite la quantité acceptable d'additif solubilisant et exclut l'utilisation d'additifs de type polymère de haute masse moléculaire très hydrosolubles mais présentant des viscosités élevées.

Enfin, dans la perspective d'une administration parentérale, il est souhaitable que l'additif solubilisant soit parfaitement toléré et se dégrade rapidement, c'est-à-dire sur une échelle de temps de quelques jours à quelques semaines. Il est également préférable que la quantité de cet additif soit la plus limitée possible.

La mise au point d'un additif de solubilisation permettant la solubilisation de principes actifs en une concentration suffisamment élevée et répondant simultanément à l'ensemble des critères rappelés précédemment est délicate et, à la connaissance des inventeurs, non atteinte à ce jour.

Plusieurs alternatives ont déjà été proposées pour tenter de suppléer au défaut de biodisponibilité des actifs faiblement hydrosolubles. Parmi celles-ci, une alternative particulièrement intéressante met en œuvre des solutions micellaires. On connaît ainsi des micelles polymériques formées de copolymères amphiphiles, par exemple de copolymères di-bloc PLGA-PEG. Dans ce mode de formulation, le principe actif est solubilisé au sein du cœur hydrophobe de PLGA des micelles.

Toutefois, cette approche présente notamment deux limitations : d'une part, un PA modérément soluble tel que par exemple un peptide de solubilité moyenne, peut être difficile à solubiliser dans le cœur hydrophobe et d'autre part, le procédé de fabrication des nanoparticules comprend une étape de solubilisation du PLGA dans un solvant hydrophobe, étape qui doit être évitée pour certains PA fragiles.

La présente invention vise précisément à proposer de nouvelles compositions particulièrement avantageuses au regard d'actifs de faible solubilité aqueuse, dans la mesure où elles permettent de donner satisfaction à l'ensemble des exigences précitées.

La présente invention décrit une composition comprenant au moins un actif de faible solubilité aqueuse, en particulier inférieure à 1 g/l d'eau pure, ledit actif y étant présent sous une forme associée de manière non covalente à des nanoparticules formées d'au moins un polymère POM de formule (II) définie ci-dessous ou l'un de ses sels pharmaceutiquement acceptables et dans laquelle ledit actif est présent à raison d'au moins 5 µmol/g de polymère POM.

Elle décrit également une composition comprenant au moins un actif de solubilité aqueuse moyenne, en particulier comprise entre 1 g/l et 30 g/l d'eau pure, ledit actif y étant présent sous une forme associée de manière non covalente à des nanoparticules formées d'au moins un polymère POM de formule (II) définie ci-dessous ou l'un de ses sels pharmaceutiquement acceptables, et dans laquelle ledit actif est présent à raison d'au moins 5 µmol/g de polymère POM.

L'invention décrit également l'utilisation de nanoparticules, telles que définies précédemment, d'au moins un polymère POM, associées de manière non covalente à un actif, en vue d'accroître la solubilisation aqueuse dudit actif.

Plus précisément, un premier objet de l'invention est une composition comprenant au moins un actif de solubilité aqueuse inférieure à 1 g/l d'eau pure, ledit actif y étant présent sous une forme associée de manière non covalente à des nanoparticules formées d'au moins un polymère POM de formule (II) suivante ou l'un de ses sels pharmaceutiquement acceptables, 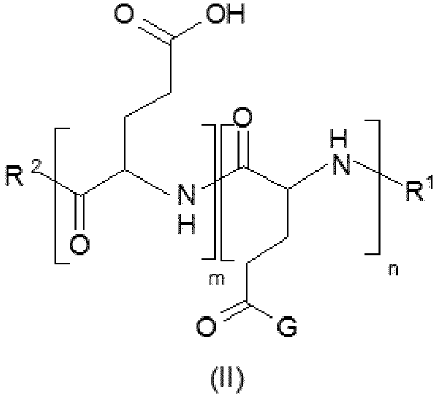 dans laquelle :
- R¹ représente un atome d'hydrogène, un groupe acyle linéaire en C₂ à C₁₀, un groupe acyle ramifié en C₃ à C₁₀, un groupe pyroglutamate ou un groupement hydrophobe G ;
- R² représente un groupe -NHR⁵ ou un résidu acide aminé terminal lié par l'azote et dont le carboxyle est éventuellement substitué par un radical alkylamino -NHR⁵ ou un alcoxy -OR⁶ ;
- R⁵ représente un atome d'hydrogène, un groupe alkyle linéaire en C₁ à C₁₀, un groupe alkyle ramifié en C₃ à C₁₀, ou un groupe benzyle ;
- R⁶ représente un atome d'hydrogène, un groupe alkyle linéaire en C₁ à C₁₀, un groupe alkyle ramifié en C₃ à C₁₀, un groupe benzyle ou un groupement G ;
- G représente un radical tocophéryl ;
- m et n sont des entiers positifs, non nuls ;
- (m+n) varie de 25 à 100 ;
- le taux de greffage molaire des groupements hydrophobes G, (n)/(n+m) varie de 10 à 21 % molaire;
et dans laquelle ledit actif est présent à raison d'au moins 5 µmol/g de POM ;
à l'exclusion d'une composition comprenant du carvédilol base sous une forme associée de manière non covalente à des nanoparticules formées d'un polymère polyglutamate greffé à 20% de vitamine E et présentant un degré de polymérisation d'environ 100, le carvédilol base étant présent à raison d'environ 248 µmol/g de polyglutamate ; et
à l'exclusion d'une composition comprenant du carvédilol base sous une forme associée de manière non covalente à des nanoparticules formées d'un polymère polyglutamate greffé à 10% de vitamine E et présentant un degré de polymérisation d'environ 100, le carvédilol base étant présent à raison d'environ 311 µmol/g de polyglutamate.

L'invention concerne encore, selon un autre de ses aspects, l'utilisation de nanoparticules d'au moins un polymère POM telles que définies précédemment, associées de manière non covalente à un actif, ledit actif présentant une solubilité aqueuse inférieure à 1 g/l d'eau pure, en vue d'accroître la solubilisation aqueuse dudit actif, ledit principe actif étant mis en œuvre à raison d'au moins 5 µmol/g de POM.

Avantageusement, une composition de l'invention permet de solubiliser les actifs considérés sous une forme liquide de faible viscosité, stable, aisément manipulable et/ou injectable à travers des aiguilles de faible diamètre. Elle présente en outre l'avantage d'être biocompatible et de ne pas nécessiter, pour être mise en œuvre, d'étape de formulation dénaturante, d'utilisation de solvant organique ou de tensioactifs.

Certes, des nanoparticules de polymère ont déjà été proposées à titre de mode d'administration par voie parentérale de divers actifs. Des nanoparticules d'un copolyaminoacide comprenant des groupements hydrophobes et des groupements hydrophiles sont ainsi proposées dans les documents WO 96/29991 et WO 03/104303 de la société Flamel Technologies. Le document WO 03/104303 divulgue plus particulièrement un polymère de type polyaminoacide comprenant des résidus aspartiques et/ou des résidus glutamiques, avec au moins une partie de ces résidus comportant au moins un motif alpha-tocophérol. Ces homopolyaminoacides « modifiés hydrophobes » forment spontanément dans l'eau une suspension colloïdale de nanoparticules, lesquelles sont aptes à s'associer aisément en suspension aqueuse à pH 7,4, avec au moins une protéine active. La demande WO 2005/051416 propose pour sa part des polyaminoacides biodégradables, transformables en nano- ou micro-particules colloïdales de vectorisation aptes à s'associer réversiblement à des principes actifs et à assurer une libération prolongée desdits principes actifs.

Le document WO 2008/025425 divulgue des microparticules de polymères amphiphiles, en particulier de polyglutamates greffés par l'alpha-tocophérol, associées de manière non covalente avec un principe actif tel que l'ITFN-α-2b.

Toutefois, ces documents ne sont aucunement concernés par le problème de défaut de solubilité aqueuse posé par certains actifs. Qui plus est, leur enseignement n'est pas de nature à guider l'homme du métier, vers le choix d'une structure spécifique de polymère pour répondre à ce problème.

Ainsi, la présente invention résulte notamment de la découverte des inventeurs que le pouvoir solubilisant de polymères amphiphiles spécifiques porteurs de groupements hydrophobes, notamment ceux de la famille des polyglutamates porteurs de motifs vitamine E pendants, dépend de façon singulière du degré de substitution en groupements hydrophobes. Plus précisément, les inventeurs ont constaté que le pouvoir solubilisant n'est pas proportionnel au taux de groupements hydrophobes portés par le polymère. Le pouvoir solubilisant croît tout d'abord rapidement avec le taux de groupements hydrophobes, puis atteint un plateau, voire passe par un maximum puis décroît lentement. Sans vouloir être limité par la théorie, on peut supposer que le pouvoir solubilisant résulte d'un compromis entre d'une part l'abondance des groupes hydrophobes et d'autre part leur accessibilité.

Comme indiqué précédemment, le pouvoir solubilisant n'est pas le seul paramètre à prendre en compte pour permettre une administration optimale. Les inventeurs ont ainsi constaté en outre, qu'afin d'abaisser la viscosité de la suspension, il convenait d'augmenter la proportion de groupements hydrophobes et de privilégier les degrés de polymérisation faibles. Là encore, sans vouloir être lié par la théorie, on peut supposer que les polymères de petite taille et fortement substitués par des groupes hydrophobes s'auto-associent en particules de plus grande densité.

Les inventeurs ont ainsi découvert qu'il était possible d'améliorer de façon spectaculaire la solubilisation aqueuse de principes actifs, en particulier d'actifs faiblement ou moyennement hydrosolubles, pour atteindre une concentration suffisante en principes actifs solubilisés, afin de permettre notamment leur administration dans une dose unitaire de taille acceptable pour le patient, ceci à travers l'utilisation de nanoparticules d'au moins un polymère POM de formule (II) définie par la suite, présentant en particulier un degré de polymérisation compris entre 25 et 250, plus particulièrement entre 40 et 100 et un taux de greffage en groupements hydrophobes variant de 4 à 25 % molaire, voire de 5 à 22 % molaire.

De manière avantageuse, la formulation considérée selon l'invention peut être en outre apte à assurer un profil de libération de cet actif régulé en fonction du temps.

Dans une réalisation préférée de l'invention, les inventeurs ont découvert qu'avantageusement, le degré de polymérisation du polymère devait être compris entre 25 et 100 et le taux de greffage en groupements hydrophobes compris entre 5 et 25 %.

Sans vouloir être lié par la théorie, il semble qu'un degré de polymérisation trop faible ne permet pas de contrôler aisément le nombre de greffons de groupements hydrophobes par chaines.

A l'inverse, un degré de polymérisation trop important, en particulier strictement supérieur à 100, conduit à une suspension colloïdale dont la viscosité devient importante même pour des concentrations limitées, ce qui doit être évité pour l'administration parentérale du produit.

En ce qui concerne le taux de greffage, il est du mérite des inventeurs d'avoir constaté qu'un tel taux, lorsqu'il varie de 5 à 25 %, permet d'accéder à un optimum de pouvoir solubilisant du polymère POM correspondant.

Ainsi, les inventeurs ont constaté qu'un taux de greffage trop important, en particulier strictement supérieur à 25 %, voire supérieur à 22 %, conduit à des nanoparticules de polymère POM ayant une stabilité colloïdale limitée dans la gamme de pH comprise entre 6,0 et 7,2, qui est la gamme de pH la plus fréquemment utilisée pour les formes pharmaceutiques.

### PRINCIPES ACTIFS

Comme mentionné précédemment, la présente invention vise à solubiliser en concentrations suffisantes des actifs de solubilité aqueuse faible ou moyenne et/ou à accroître la solubilisation aqueuse des principes actifs en général.

L'invention s'avère ainsi tout particulièrement avantageuse à l'égard d'actifs faiblement hydrosolubles.

Au sens de la présente invention, un actif de faible solubilité aqueuse est un composé possédant une solubilité inférieure à 1 g/l, notamment inférieure à 0,1 g/l, dans l'eau pure, mesurée à température ambiante, c'est-à-dire 25 °C environ.

Au sens de l'invention, une eau pure est une eau de pH proche de la neutralité (entre pH 5 et pH 8) et dénuée de tout autre composé solubilisant connu de l'homme de l'art, tels que des tensioactifs ou des polymères (PVP, PEG).

Les principes actifs considérés selon l'invention sont avantageusement des composés biologiquement actifs et qui peuvent être administrés à un organisme animal ou humain.

Selon une variante de réalisation, ces principes actifs sont non peptidiques.

D'une manière générale, un actif selon l'invention peut être toute molécule d'intérêt thérapeutique, cosmétique, prophylactique ou d'imagerie.

Avantageusement, les actifs selon l'invention sont des actifs de petite taille.

Par actifs de petite taille, on entend les molécules organiques de masse inférieure à 2.000 Da, en particulier inférieure à 1.000 Da.

Ainsi, dans le domaine pharmaceutique, les actifs de faible solubilité aqueuse selon l'invention peuvent être notamment choisis parmi les agents anticancéreux, les agents antifongiques, les stéroïdes, les agents anti-inflammatoires, les hormones sexuelles, les immunosuppresseurs, les agents antiviraux, les anesthésiques, les antiémétiques et les agents antihistaminiques.

Plus particulièrement, peuvent être cités à titre représentatif d'actifs spécifiques de faible solubilité aqueuse les dérivés du taxane tel que le paclitaxel, la nifédipine, le carvédilol, la camptothécine, la doxorubicine, le cisplatine, le 5-fluorouracile, la cyclosporine A, le PSC 833, l'amphotéricine B, l'itraconazole, le kétoconazole, la bétaméthasone, l'indométhacine, la testostérone, l'estradiol, la dexaméthasone, la prednisolone, l'acétonide de triamcinolone, la nystatine, le diazépam, l'amiodarone, le vérapamil, la simvastatine, la rapamycine et l'étoposide.

Selon un mode de réalisation particulier, l'actif considéré selon l'invention est un actif à intérêt thérapeutique.

Selon un autre mode de réalisation particulier, l'actif peut être choisi parmi le paclitaxel, le carvédilol base, la simvastatine, la nifédipine, le kétoconazole et la cyclosporine-A.

Selon la présente invention, le principe actif est présent dans une composition de l'invention à raison d'au moins 5 µmol/g de polymère POM, plus particulièrement à raison d'au moins 10 µmol/g de POM, plus particulièrement à raison d'au moins 20 µmol/g de POM, et encore plus particulièrement à raison d'au moins 100 µmol/g de POM.

### NANOPARTICULES

Le principe actif, tel que décrit précédemment, est présent dans une composition selon l'invention au moins en partie sous une forme associée de manière non covalente à des nanoparticules formées d'au moins un polymère POM de formule (II) suivante ou l'un de ses sels pharmaceutiquement acceptables, 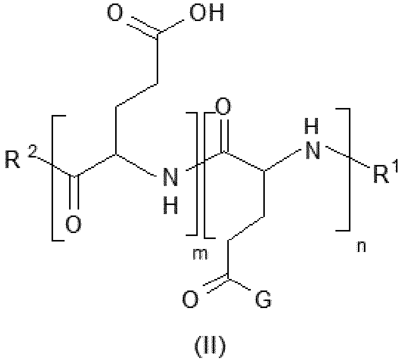 dans laquelle :
- R¹ représente un atome d'hydrogène, un groupe acyle linéaire en C₂ à C₁₀, un groupe acyle ramifié en C₃ à C₁₀, un groupe pyroglutamate ou un groupement hydrophobe G ;
- R² représente un groupe -NHR⁵ ou un résidu acide aminé terminal lié par l'azote et dont le carboxyle est éventuellement substitué par un radical alkylamino -NHR⁵ ou un alcoxy -OR⁶ ;
- R⁵ représente un atome d'hydrogène, un groupe alkyle linéaire en C₁ à C₁₀, un groupe alkyle ramifié en C₃ à C₁₀, ou un groupe benzyle ;
- R⁶ représente un atome d'hydrogène, un groupe alkyle linéraire en C₁ à C₁₀, un groupe alkyle ramifié en C₃ à C₁₀, un groupe benzyle ou un groupement G ;
- G représente un radical tocophéryl ;
- m et n sont des entiers positifs, non nuls ;
- (m+n) varie de 25 à 100, en particulier de 40 à 100, plus particulièrement de 40 à 100, et de préférence de 50 à 100 ;
- le taux de greffage molaire des groupements hydrophobes G, (n)/(n+m) varie de 10 à 21 % ;
l'enchaînement des monomères de ladite formule générale II pouvant être aléatoire, de type monobloc ou multibloc, de préférence étant aléatoire.

Selon une variante de l'invention, conviennent tout particulièrement à l'invention, à titre de polymère POM des polymères de formule II, dont le taux de greffage varie entre 10 et 21 % molaire et dont le DP est compris entre 50 et 150.

Il peut notamment s'agir d'un polyglutamate ayant un degré de polymérisation d'environ 100, et greffé à 15 % ou à 20 % en vitamine E.

La formule générale (II) décrite ci-dessus ne doit pas être interprétée comme représentant uniquement des copolymères séquencés (ou blocs), mais également des copolymères aléatoires ou des copolymères multiblocs.

On entend par « sels pharmaceutiquement acceptables » du polymère selon l'invention l'ensemble des polymères avec les contre-ions associés aux fonctions ionisées du polymère. Il est aussi envisageable, pour certaines structures où il y a co-existence des charges positives et négatives qu'il y ait une neutralisation totale ou partielle des charges.

Les termes « association » ou « associé » employés pour qualifier les relations entre un ou plusieurs principes actifs et le polymère POM selon l'invention, signifient que le ou les principes actifs sont associés au(x) polymère(s) POM par des interactions physiques non covalentes, notamment des interactions hydrophobes, et/ou des interactions électrostatiques et/ou des liaisons hydrogène et/ou via une encapsulation stérique par les polymères POM.

Cette association relève généralement d'interactions hydrophobes et/ou électrostatiques réalisées par les motifs du polymère POM, notamment hydrophobes ou ionisés, aptes à générer ce type d'interaction.

Les polymères POM considérés selon l'invention sont en particulier aptes à former spontanément, lorsqu'ils sont mis en dispersion dans un milieu aqueux et notamment l'eau, des nanoparticules.

D'une manière générale, la taille des nanoparticules varie de 1 à 1.000 nm, en particulier de 5 à 500 nm, notamment de 10 à 300 nm et plus particulièrement de 10 à 100 nm.

La taille des nanoparticules de POM est évaluée par le diamètre hydrodynamique moyen de ces particules. La mesure est effectuée par diffusion quasi élastique de la lumière avec un appareil CGS-3 de ALV. A cette fin, la suspension de POM est concentrée à 0,5 mg/ml dans un milieu salin tel que NaCl 0,15 M après un temps de repos suffisant pour atteindre l'équilibre.

Avantageusement, le polymère POM est biodégradable.

Selon un mode de réalisation particulier, un polymère POM convenant à l'invention, peut être porteur d'un ou plusieurs greffons de type polyalkylène glycol lié(s) à une unité glutamate le constituant. De préférence, le polyalkylène glycol est un polyéthylène glycol et plus particulièrement mis en œuvre avec un pourcentage molaire de greffage de polyéthylène glycol variant de 1 à 30 %.

Il convient en outre de noter que les fonctions résiduelles carboxyliques du polyglutamate modifié selon l'invention sont soit neutres (forme COOH), soit ionisées (anion COO⁻), selon le pH et la composition. On parlera donc indifféremment i) de résidu glutamate ou de résidu acide glutamique, ii) d'acide polyglutamique ou de polyglutamate.

Le couplage des groupements cationiques et éventuellement neutres avec une fonction acide du polymère POM de formule (II) est réalisé simultanément dans une deuxième étape en présence d'un chloroformiate comme agent de couplage et dans un solvant approprié tel que le diméthylformamide, la *N*-méthylpyrrolidone (NMP) ou le diméthylsulfoxide (DMSO).

Dans le cas où le groupement cationique contient deux fonctions amines non différenciées chimiquement (*e.g.* diamine linéaire), il peut être introduit sous une forme dans laquelle une des deux fonctions est protégée. Une dernière étape de clivage du groupement protecteur est alors ajoutée.

La chimie de polymérisation et les réactions de couplage des groupements sont classiques et bien connues de l'homme de l'art (voir par exemples les brevets ou demandes de brevet de la demanderesse cités ci-dessus).

### Association du POM à un actif

Les techniques d'association d'un ou de plusieurs principes actifs aux polymère POM selon l'invention sont similaires à celles décrites notamment dans le brevet US 6,630,171.

Les principes actifs peuvent s'associer spontanément au polymère POM tel que décrit précédemment.

Cette association est purement physique et n'implique pas de création de liaison covalente entre le principe actif et le polymère.

Il n'est pas prévu d'étape de réticulation chimique des particules obtenues. L'absence de réticulation chimique permet d'éviter la dégradation chimique du principe actif lors de l'étape de réticulation des particules contenant le principe actif. Une telle réticulation chimique est en effet généralement conduite par activation d'entités polymérisables et met en jeu des agents potentiellement dénaturants tels que les rayonnements UV ou le glutaraldéhyde.

L'association selon l'invention du principe actif et du polymère POM peut notamment s'effectuer selon les modes suivants.

Dans un premier mode, le principe actif est dissout dans une solution aqueuse et mélangé à une suspension aqueuse du polymère POM.

Dans un deuxième mode, le principe actif sous forme de poudre est dispersé dans une suspension aqueuse du polymère POM et l'ensemble est agité jusqu'à obtention d'une suspension limpide homogène.

Dans un troisième mode, le polymère POM est introduit sous forme de poudre dans une dispersion ou une solution aqueuse du principe actif.

Dans un quatrième mode, le principe actif et/ou le polymère est/sont dissout(s) dans une solution contenant un solvant organique miscible à l'eau tel que l'éthanol ou l'isopropanol. On procède alors comme selon les modes 1 à 3 ci-dessus. Eventuellement, ce solvant peut être éliminé par dialyse ou toute autre technique connue de l'homme de l'art.

Pour l'ensemble de ces modes, il peut être avantageux de faciliter l'interaction entre le principe actif et le polymère POM à l'aide d'ultrasons ou d'une élévation de température.

Pour des raisons évidentes, le rapport pondéral principe actif/polymère POM est susceptible de varier significativement en fonction de la dose en principe actif considérée.

Plus particulièrement, ce rapport peut varier entre 0,1 et 300 % en poids, entre 1 et 100 % en poids ou entre 5 et 80 % en poids.

### MICROPARTICULES

Selon un mode de réalisation particulier, les nanoparticules associées de manière non covalente audit principe actif peuvent être mises en œuvre dans une composition selon l'invention, sous la forme de microparticules.

Selon un premier mode de réalisation, ces microparticules peuvent être obtenues par agglomération de nanoparticules de l'invention selon les méthodes connues de l'homme de l'art, par exemple, de manière illustrative et non limitative, par floculation, atomisation, lyophilisation ou coacervation.

Selon un autre mode de réalisation, les microparticules possèdent un cœur contenant lesdites nanoparticules et au moins une couche d'enrobage conditionnant un profil de libération régulé dudit principe actif en fonction du pH, ladite couche d'enrobage étant formée d'un matériau comprenant au moins un polymère A possédant une valeur de pH de solubilisation comprise dans la plage de pH de 5 à 7 associé à au moins un composé B hydrophobe.

La libération contrôlée en fonction du pH des nanoparticules à partir des microparticules est assurée par l'enrobage entourant le cœur de chaque particule réservoir. Cet enrobage est conçu de manière à libérer le principe actif et le polymère POM à des sites bien spécifiques du tractus gastro-intestinal correspondant par exemple aux fenêtres d'absorption du principe actif dans le tractus gastro-intestinal.

De par la nature de cet enrobage, les microparticules considérées selon la présente invention peuvent ainsi avantageusement présenter un double mécanisme de libération en fonction du temps et du pH.

Sous cette expression, il est entendu qu'elles possèdent les deux spécificités suivantes. En-deçà de la valeur de pH de solubilisation du polymère A formant l'enrobage de ces microparticules, elles ne libèrent qu'une quantité très limitée de nanoparticules. En revanche, lorsqu'elles sont présentes dans l'intestin ou un milieu assimilable, assurent une libération effective des nanoparticules. Cette libération peut être alors réalisée avantageusement en moins de 24 heures, en particulier en moins de 12 heures, notamment en moins de 6 heures, en particulier moins de 2 heures voire en moins de 1 heure.

Dans le cas de principes actifs ayant une fenêtre d'absorption très étroite, par exemple limitée au duodénum ou aux plaques de Peyer, le temps de libération des nanoparticules est inférieur à 2 heures et de préférence inférieur à 1 heure.

Ainsi une composition selon l'invention est propice à libérer dans un premier temps le principe actif associé aux nanoparticules de polymère(s) POM puis à dissocier dans un second temps le principe actif desdites nanoparticules.

La taille des microparticules considérées selon cette variante de l'invention est avantageusement inférieure à 2.000 µm, en particulier varie de 100 à 1.000 µm, en particulier de 100 à 800 µm et notamment de 100 à 500 µm.

Au sens de l'invention, la taille des particules est exprimée en diamètre moyen en volume D_{4,3} mesuré par granulométrie laser à l'aide d'un appareil Mastersizer 2000 de Malvern Instrument équipé du module voie sèche Sirocco 2000.

Selon cette variante de réalisation, l'enrobage des nanoparticules peut être formé d'un matériau composite obtenu par mélange de :
- au moins un composé A possédant une valeur de pH de solubilisation comprise dans la plage de pH de 5 à 7 ;
- au moins un composé B hydrophobe ;
- et éventuellement au moins un agent plastifiant et/ou autres excipients conventionnels.

### Polymère A

Au sens de la présente invention, la valeur de pH de solubilisation du polymère A est une valeur de pH du milieu physiologique ou du milieu *in vitro* modèle en-deçà de laquelle le polymère se trouve dans un état insoluble et au-delà de laquelle ce même polymère A se trouve à un état soluble.

Pour des raisons évidentes, cette valeur de pH est spécifique à un polymère donné et directement liée à ses caractéristiques physico-chimiques intrinsèques, telles que sa nature chimique et sa longueur de chaîne.

A titre illustratif et non limitatif des polymères A convenant à l'invention, on peut notamment citer :
- le(s) copolymère(s) d'acide méthacrylique et de méthacrylate de méthyle,
- le(s) copolymère(s) d'acide méthacrylique et d'acrylate d'éthyle,
- les dérivés cellulosiques tels que :
   ∘ l'acétate phtalate de cellulose (CAP),
   ∘ l'acétate succinate de cellulose (CAS),
   ∘ l'acétate trimellitate de cellulose (CAT),
   ∘ le phtalate d'hydroxypropylméthylcellulose (ou hypromellose phtalate) (HPMCP),
   ∘ l'acétate succinate d'hydroxypropylméthylcellulose (ou hypromellose acétate succinate) (HPMCAS),
- la gomme shellac,
- l'acétate phtalate de polyvinyle (PVAP),
- et leurs mélanges.

Selon un mode préféré de l'invention, ce polymère A est choisi parmi le(s) copolymère(s) d'acide méthacrylique et de méthacrylate de méthyle, le(s) copolymère(s) d'acide méthacrylique et d'acrylate d'éthyle et leurs mélanges.

Comme précisé précédemment, le polymère A considéré selon l'invention possède un profil de solubilité différent selon qu'il est confronté à une valeur de pH supérieure ou inférieure à sa valeur de pH de solubilisation.

Au sens de l'invention, le polymère A est généralement insoluble à une valeur de pH inférieure à sa valeur de pH de solubilisation et en revanche soluble à une valeur de pH supérieure à sa valeur de pH de solubilisation.

Par exemple, il peut s'agir d'un polymère dont la valeur de pH de solubilisation est de :
- 5,0 à l'image par exemple du phtalate d'hydroxypropylméthylcellulose et notamment celui commercialisé sous la dénomination HP-50 par Shin-Etsu,
- 5,5 à l'image par exemple du phtalate d'hydroxypropylméthylcellulose et notamment celui commercialisé sous la dénomination HP-55 par Shin-Etsu ou du copolymère d'acide méthacrylique et d'acrylate d'éthyle 1 :1 et notamment celui commercialisé sous la dénomination Eudragit L100-55 de Evonik,
- 6,0 à l'image par exemple d'un copolymère d'acide méthacrylique et de méthacrylate de méthyle 1:1 et notamment celui commercialisé sous la dénomination Eudragit L100 de Evonik,
- 7,0 comme par exemple un copolymère d'acide méthacrylique et de méthacrylate de méthyle 1 :2 et notamment celui commercialisé sous la dénomination Eudragit S100 de Evonik.

L'ensemble de ces polymères est soluble à une valeur de pH supérieure à son pH de solubilisation.

L'enrobage est avantageusement composé de 25 à 90 %, en particulier de 30 à 80 %, notamment de 35 à 70 %, voire de 40 à 60 % en poids de polymère(s) A par rapport à son poids total.

Plus préférentiellement, le polymère A est un copolymère d'acide méthacrylique et d'acrylate d'éthyle 1:1.

### Composé B hydrophobe

Selon une première variante, le composé B peut être sélectionné parmi les produits cristallisés à l'état solide et ayant une température de fusion T_{fb} ≥ 40 °C, de préférence T_{fb} ≥ 50 °C, et plus préférentiellement encore 40°C ≤ T_{fb} ≤ 90 °C.

Plus préférentiellement, ce composé est alors choisi parmi le groupe de produits suivants :
- cires végétales prises seules ou en mélange entre-elles, telles que celles commercialisées sous les marques DYNASAN P60; DYNASAN 116, entre autres ;
- huiles végétales hydrogénées prises seules ou en mélange entre-elles; de préférence choisies dans le groupe comprenant : l'huile de coton hydrogénée, l'huile de soja hydrogénée, l'huile de palme hydrogénée et leurs mélanges ;
- mono et/ou di et/ou tri esters du glycérol et d'au moins un acide gras, de préférence l'acide béhénique, pris à eux seuls ou en mélange entre eux ;
- et leurs mélanges.

Selon ce mode de réalisation, le rapport pondéral B/A peut varier entre 0,2 et 1,5 et de préférence entre 0,45 et 1.

Plus préférentiellement, le composé B est l'huile de coton hydrogénée.

Des microparticules formées d'un tel enrobage sont notamment décrites dans le document WO 03/30878.

Selon une seconde variante, le composé B peut être un polymère insoluble dans les liquides du tube digestif.

Ce polymère insoluble dans les liquides du tube digestif ou encore les fluides gastro-intestinaux est plus particulièrement sélectionné parmi :
- les dérivés non hydrosolubles de la cellulose,
- les dérivés non hydrosolubles de (co)polymères (méth)acryliques,
- et leurs mélanges.

Plus préférentiellement, il peut être choisi parmi l'éthylcellulose, et/ou des dérivés, par exemple, ceux commercialisés sous la dénomination Ethocel®, l'acétate butyrate de cellulose, l'acétate de cellulose, les copolymères d'ammonio (méth)acrylate (copolymère d'acrylate d'éthyle, de méthacrylate de méthyle et de méthacrylate de triméthylammonio éthyle) de type « A » ou de type « B », notamment ceux commercialisés sous les dénominations Eudragit® RL et Eudragit® RS, les esters d'acides poly(méth)acryliques, notamment ceux commercialisés sous la dénomination Eudragit® NE et leurs mélanges.

Conviennent tout particulièrement à l'invention l'éthylcellulose, l'acétate butyrate de cellulose et les copolymères d'ammonio (méth)acrylate, notamment ceux commercialisés sous la dénomination Eudragit RS® et Eudragit RL®.

L'enrobage des microparticules contient alors de 10 % à 75 %, et peut contenir de préférence de 15 % à 60 %, plus préférentiellement de 20 % à 55 %, voire de 25 à 55 % en poids, et plus particulièrement encore de 30 à 50 % de polymère(s) A par rapport à son poids total.

Avantageusement, l'enrobage peut être alors formé, selon ce mode de réalisation, à partir d'un mélange des deux catégories de polymères A et B dans un rapport pondéral polymère(s) B/polymère(s) A supérieur à 0,25, en particulier supérieur ou égal à 0,3, en particulier supérieur ou égal à 0,4, notamment supérieur ou égal à 0,5, voire supérieur ou égal à 0,75.

Selon une autre variante de réalisation, le rapport polymère(s) A/polymère(s) B est en outre inférieur à 8, notamment inférieur à 4, voire inférieur à 2 et plus particulièrement inférieur à 1,5.

A titre représentatif des mélanges polymères A et B convenant tout particulièrement à l'invention, peuvent notamment être cités les mélanges d'éthylcellulose, d'acétate butyrate de cellulose ou de copolymère d'ammonio(méth)acrylate de type A ou B avec au moins un copolymère d'acide méthacrylique et d'acrylate d'éthyle ou un copolymère d'acide méthacrylique et de méthacrylate de méthyle ou un de leurs mélanges.

Outre les deux types de composés A et B précités, l'enrobage des nanoparticules selon l'invention peut comprendre au moins un agent plastifiant.

### Agent plastifiant

Cet agent plastifiant peut être notamment choisi parmi :
- le glycérol et ses esters, et de préférence parmi les glycérides acétylés, glycéryl-mono-stéarate, glycéryl-triacétate, glycéryl-tributyrate,
- les phtalates, et de préférence parmi les dibutylphtalate, diéthylphtalate, diméthylphtalate, dioctylphtalate,
- les citrates, et de préférence parmi les acétyltributylcitrate, acétyltriéthylcitrate, tributylcitrate, triéthylcitrate,
- les sébaçates, et de préférence parmi les diéthylsébaçate, dibutylsébaçate,
- les adipates,
- les azélates,
- les benzoates,
- le chlorobutanol,
- les polyéthylènes glycols,
- les huiles végétales,
- les fumarates, de préférence le diéthylfumarate,
- les malates, de préférence le diéthylmalate,
- les oxalates, de préférence le diéthyloxalate,
- les succinates ; de préférence le dibutylsuccinate,
- les butyrates,
- les esters de l'alcool cétylique,
- les malonates, de préférence le diéthylmalonate,
- l'huile de ricin,
- et leurs mélanges.

En particulier, l'enrobage peut comprendre moins de 30 % en poids, de préférence de 1 % à 25 % en poids, et, plus préférentiellement encore, de 5 % à 20 % en poids d'agent(s) plastifiant(s) par rapport à son poids total.

La formation des microparticules selon cette variante de l'invention peut être réalisée par toute technique conventionnelle propice à la formation d'une capsule réservoir dont le cœur est formé en tout ou partie d'au moins un principe actif associé de manière non covalente à des nanoparticules de polymère POM, notamment telles que définies ci-dessus.

De manière préférentielle, les microparticules sont formées par pulvérisation des composés A et B et si présent(s) les autres ingrédients dont le (ou les) plastifiant(s) à l'état généralement de solutés. Ce milieu solvant contient généralement des solvants organiques mélangés ou non avec de l'eau. L'enrobage ainsi formé s'avère homogène en termes de composition par opposition à un enrobage formé à partir d'une dispersion de ces mêmes polymères, dans un liquide majoritairement aqueux.

Selon une variante de réalisation préférée, la solution pulvérisée contient moins de 40 % en poids d'eau, en particulier moins de 30 % en poids d'eau et plus particulièrement moins de 25 % en poids d'eau.

Selon une autre variante de réalisation, les nanoparticules associées de manière non covalente au principe actif, peuvent être mises en œuvre sous une forme supportée, de type microparticules, en particulier sur un substrat neutre à l'aide d'un ou plusieurs liants et avec un ou plusieurs excipients conventionnels.

Ces microparticules, présentes sous une forme supportée peuvent être ultérieurement enrobées par une ou plusieurs couches d'enrobage, telle(s) que décrite(s) précédemment.

Dans le cas où l'on souhaite déposer le mélange PA/POM sur un substrat neutre de type sphère neutre, on peut procéder de la façon suivante :
Au mélange homogène de principe actif et de POM, on ajoute un liant conventionnel destiné à assurer la cohésion de la couche déposée sur le cœur neutre.

De tels liants sont notamment proposés dans Khankari R.K. et al., Binders and Solvents in Handbook of Pharmaceutical Granulation Technology, Dilip M. Parikh ed., Marcel Dekker Inc., New York, 1997.

Conviennent tout particulièrement à l'invention à titre de liant : l'hydroxypropylcellulose (HPC), la polyvinylpyrrolidone (PVP), la méthylcellulose (MC) et l'hydroxypropylméthylcellulose (HPMC).

Le dépôt du mélange correspondant s'effectue alors par les techniques classiques connues de l'homme de l'art. Il peut notamment s'agir d'une pulvérisation de la suspension colloïdale des nanoparticules chargées en principes actifs et contenant le liant, et éventuellement d'autres composés, sur le support dans un lit d'air fluidisé.

Sans que cela ne soit limitatif, une composition selon l'invention peut par exemple contenir, outre les nanoparticules associées au principe actif et les excipients conventionnels, du sucrose et/ou du dextrose et/ou du lactose, ou bien encore une microparticule d'un substrat inerte tel que la cellulose servant de support pour lesdites nanoparticules.

Ainsi, dans un premier mode de réalisation préféré de cette variante, une composition selon l'invention peut comprendre des granulés contenant le POM, le principe actif, un ou plusieurs liants assurant la cohésion du granulé et divers excipients connus de l'homme de l'art.

Un enrobage peut être ensuite déposé sur ce granulé par toute technique connue de l'homme de l'art, et avantageusement par spray coating, conduisant à la formation de microparticules telles que décrites précédemment.

La composition pondérale d'une microparticule conforme à ce mode de réalisation est la suivante :
- la teneur pondérale en nanoparticules chargées en principe actif dans le cœur est comprise entre 0,1 et 80 %, de préférence entre 2 et 70 % de préférence encore entre 10 et 60 % ;
- la teneur pondérale en liant dans le cœur est comprise entre 0,5 et 40 %, de préférence entre 2 et 25 % ;
- la teneur pondérale de l'enrobage dans la microparticule est comprise entre 5 et 50 %, de préférence entre 15 et 35 %.

Dans un deuxième mode de réalisation préféré de cette variante, une composition selon l'invention peut comprendre des cœurs neutres autour desquels on a déposé une couche contenant le principe actif, les nanoparticules POM, un liant assurant la cohésion de cette couche et éventuellement différents excipients connus de l'homme de l'art, par exemple le sucrose, le tréhalose et le mannitol. Le cœur neutre peut être une particule de cellulose ou de sucre ou tout composé inerte organique ou salin qui se prête à l'enrobage.

Les cœurs neutres ainsi recouverts peuvent alors être enrobés par au moins une couche d'enrobage pour former des microparticules telles que décrites précédemment.

La composition pondérale d'une particule selon ce mode de réalisation est alors la suivante :
- la teneur pondérale en nanoparticules chargées en principe actif dans le cœur est comprise entre 0,1 et 80 %, de préférence entre 2 et 70 % de préférence encore entre 10 et 60 % ;
- la teneur pondérale de cœur neutre dans le cœur des microparticules est comprise entre 5 et 50 %, de préférence entre 10 et 30 % ;
- la teneur pondérale en liant dans le cœur des microparticules est comprise entre 0,5 et 40 %, de préférence entre 2 et 25 % ;
- la teneur pondérale de l'enrobage dans la microparticule est comprise entre 5 et 50 %, de préférence entre 15 et 35 %.

La présente invention concerne, en outre, des nouvelles préparations pharmaceutiques, phytosanitaires, alimentaires, cosmétiques ou diététiques élaborées à partir des compositions selon l'invention.

La composition selon l'invention peut ainsi se présenter sous la forme d'une poudre, d'une suspension, d'un comprimé ou d'une gélule.

La composition selon l'invention peut notamment être destinée à la préparation de médicaments.

Selon une variante de réalisation, une composition selon l'invention peut comprendre au moins deux types de nanoparticules, se différenciant par la nature du principe actif et/ou du POM associé audit principe actif.

Selon encore une autre variante, pouvant être combinée à la variante précédente, dans le cas où les nanoparticules sont mises en œuvre sous la forme de microparticules, tel que décrit ci-dessus, une composition selon l'invention peut réunir au moins deux types de microparticules se différenciant l'une de l'autre de par la nature de leur couche d'enrobage et/ou du principe actif qu'elles incorporent.

L'invention sera mieux expliquée par les exemples ci-après, donnés uniquement à titre d'illustration.

### EXEMPLES

### Exemple 1

### Préparation d'une solution de paclitaxel dans un tampon aqueux (référence).

0,49 mg de paclitaxel (Bioxel) sont introduits dans une fiole jaugée de 2 litres. La fiole est complétée jusqu'au trait de jauge par un tampon phosphate 0,05 M à pH 7,0. La préparation est agitée à l'aide d'un barreau aimanté à 37 °C. Après 2,5 h d'agitation magnétique à 37 °C, il reste quelques grains de poudre non dissous.

La préparation est alors placée pendant 60 min dans un bain à ultrasons à température ambiante. Quelques grains de poudre sont encore non dissous après ultrasons.

La solubilité du paclitaxel dans un tampon aqueux à pH 7,0 est inférieure à 0,25 µg/ml, c'est-à-dire inférieure à 0,3 µmol/l.

### Exemple 2

### Préparation de formulations aqueuses de paclitaxel avec un polyglutamate de sodium de degré de polymérisation (DP) = 100 et greffé à 20 % de vitamine E, concentrées à 20 mg/ml en polymère.

### Formulation 1

4,99 mg de paclitaxel (Bioxel) sont introduits dans un pilulier. 10 ml de solution aqueuse de polyglutamate de DP = 100 greffé à 20 % en vitamine E, à pH 7,0 et concentrée à 20 mg/ml, sont ajoutés. La préparation est placée dans un bain à ultrasons à température ambiante pendant 90 minutes. Après ultrasons, une solution parfaitement limpide est obtenue.

### Formulation 2

10,33 mg de paclitaxel (Bioxel) sont introduits dans un pilulier. 10 ml de solution aqueuse de polymère polyglutamate de DP = 100 greffé à 20 % en vitamine E, à pH = 7,0 et concentrée à 20 mg/ml, sont ajoutés. La préparation est placée dans un bain à ultrasons à température ambiante pendant 2,5 h. Après ultrasons, quelques grains de poudre de paclitaxel ne sont pas dissous.

### Formulation 3

7 mg de paclitaxel (Bioxel) sont introduits dans un pilulier. 10 ml de solution aqueuse de polymère polyglutamate greffé à 20 % en vitamine E, à pH 7,0 et concentrée à 20 mg/ml, sont ajoutés. La préparation est placée dans un bain à ultrasons à température ambiante pendant 2,5 h. Après ultrasons, une solution parfaitement limpide est obtenue.

La solubilité du paclitaxel dans une solution aqueuse à 20 mg/ml de polyglutamate de DP = 100 greffé à 20 % en vitamine E est de l'ordre de 0,8 mg/ml soit de 47 µmol de paclitaxel par gramme de polymère POM.

La solubilité du paclitaxel est ainsi considérablement améliorée par la mise en œuvre des nanoparticules de polymère POM décrites précédemment.

### Exemple 3

### Préparation de formulations aqueuses de paclitaxel avec un polyglutamate de sodium de DP = 100 greffé à 20 % de vitamine E. concentrées à 90 mg/ml en polymère.

### Formulation 1

21,21 mg de paclitaxel (Bioxel) sont introduits dans un pilulier. 10 ml de solution aqueuse de polymère polyglutamate de DP = 100 greffé à 20 % en vitamine E, à pH = 7,0 et concentrée à 90 mg/ml, sont ajoutés. La préparation est placée dans un bain à ultrasons à température ambiante pendant 60 min. Après ultrasons, une solution parfaitement limpide est obtenue. Toute la poudre de paclitaxel est solubilisée.

### Formulation 2

39,68 mg de paclitaxel (Bioxel) sont introduits dans un pilulier. 10 ml de solution aqueuse de polymère polyglutamate de DP = 100 greffé à 20 % en vitamine E, à pH = 7,0 et concentrée à 90 mg/ml, sont ajoutés. La préparation est placée dans un bain à ultrasons à température ambiante pendant 5 h. Après ultrasons, quelques grains de poudre de paclitaxel ne sont pas dissous.

### Formulation 3

35,05 mg de paclitaxel (Bioxel) sont introduits dans un pilulier. 10 ml de solution aqueuse de polymère polyglutamate greffé à 20 % en vitamine E, à pH = 7,0 et concentrée à 90 mg/ml, sont ajoutés. La préparation est placée dans un bain à ultrasons à température ambiante pendant 5 h. Après ultrasons, quelques grains de poudre de paclitaxel ne sont pas dissous.

La solubilité du paclitaxel dans une solution aqueuse à 90 mg/ml de polyglutamate greffé à 20 % en vitamine E est comprise entre 3,5 mg/ml et 4 mg/ml soit de 49 µmol de paclitaxel par gramme de polymère POM.

### Exemple 4

### Préparation de formulations aqueuses de Paclitaxel solubilisé par différents polymères POM (formulations contenant 20 g/l en polymère).

Dans cet exemple les polymères POM utilisés sont des polyglutamates de sodium ayant un DP = 100 et des taux de greffages en vitamine E variables.

La préparation des formulations dérive du protocole décrit dans l'exemple 2. La valeur de solubilité est estimée par approche successive en essayant de dissoudre de 5 à 10 mg de paclitaxel dans 10 ml de solution aqueuse de polymère POM à pH 7,0 et concentrée à 20 g/l en polymère. La préparation est placée dans un bain à ultrasons à température ambiante pendant 90 minutes. La préparation est ensuite laissée à agiter à température ambiante pendant une nuit. L'aspect de la solution est alors vérifié visuellement pour voir si la poudre de paclitaxel introduite est entièrement solubilisée ou s'il reste des cristaux de paclitaxel résiduels.

**Tableau 1 - solubilisation de paclitaxel par une solution aqueuse contenant 20 g/l de polyglutamate de sodium de DP = 100, greffé avec 5 à 20 % de vitamine E (VE), à pH = 7,0, à température ambiante.**

| % VE sur polymère | Concentration totale d'actif solubilisée pour 20 g/l de polymère (mol/l) | Actif solubilisé en µmol par g de polymère |
|---|---|---|
| 5 | 2,3.10⁻⁴ | 12 |
| 10 | 5,9.10⁻⁴ | 29 |
| 15 | 9,4.10⁻⁴ | 47 |
| 20 | 9,4.10⁻⁴ | 47 |

La solubilité de l'actif mesurée sans polymère dans des conditions analogues dans l'exemple 1 est de 3.10⁻⁷ mol/l. Le tableau 1 montre donc que la présence de polymère POM à 20 g/l permet d'augmenter la solubilité du paclitaxel d'un facteur 500 à 3.000 selon les polymères.

Le tableau 1 montre clairement que pour un polymère de DP = 100 un maximum de pouvoir solubilisant est obtenu pour les polymères ayant entre 15 et 20 % de vitamine E et c'est donc pour ces polymères qu'on observe la plus grande quantité d'actif solubilisé.

### Exemple 5

### Préparation de formulations aqueuses de Paclitaxel solubilisé par différents polymères POM (formulations contenant de fortes concentrations en polymère)

Dans cet exemple les polymères POM utilisés sont des polyglutamates de sodium ayant un degré de polymérisation DP = 100 et des taux de greffages en vitamine E variables. La solution de polymère POM est utilisée à une haute concentration de façon à solubiliser le plus possible d'actif. Cependant de façon à ce que la solution soit facilement manipulable on maintient une viscosité de la solution < 100 mPa.s (à 20°C) ce qui limite donc la concentration en polymère de façon différente selon les polymères.

La préparation des formulations dérive du protocole décrit dans l'exemple 3. La valeur de solubilité est estimée par approche successive en essayant de dissoudre de 5 à 15 mg de paclitaxel dans 2 ml de solution aqueuse concentrée de polymère POM à pH 7,0. La préparation est placée dans un bain à ultrasons à température ambiante pendant 90 minutes. Dans le cas où il reste des insolubles visibles à l'oeil nu, la solution est replacée pendant 90 minutes supplémentaires dans le bain à ultrasons. La préparation est ensuite laissée à agiter à température ambiante pendant une nuit. L'aspect de la solution est alors vérifié visuellement pour voir si la poudre de paclitaxel introduite est entièrement solubilisée ou s'il reste des cristaux de paclitaxel résiduels.

**Tableau 2 - solubilisation de paclitaxel par une solution aqueuse concentrée de polyglutamate de sodium de DP = 100, greffé avec 5 à 30 % de vitamine E (VE), à pH = 7,0, à température ambiante.**

| % VE sur polymère (%) | Concentration de la solution de polymère (mg/ml) | Concentration totale d'actif solubilisée (mol/l) | Actif solubilisé en µmol par g de polymère |
|---|---|---|---|
| 10 | 52,8 | 1,5.10⁻³ | 29 |
| 15 | 84,6 | 3,5.10⁻³ | 42 |
| 20 | 156 | 6,4.10⁻³ | 41 |
| 30 | 53,5 | < 1,4.10⁻³ | < 26 |

Le tableau 2 montre que les polymères de DP = 100 contenant entre 15 et 20 % de vitamine E permettent de monter fortement la concentration en polymère tout en maintenant une viscosité acceptable et permettent donc d'obtenir des concentrations d'actif solubilisé importantes (> 3, 10⁻³ mol/l). Rappelons que la solubilité de l'actif mesurée sans polymère dans des conditions analogues dans l'exemple 1 est de 3.10⁻⁷ mol/l soit plus de 10.000 fois inférieure.

### Exemple 6

### Préparation de formulations aqueuses de simvastatine et nifedipine solubilisé par un polyglutamate de sodium de DP = 100 greffé à 20 % de vitamine E (formulations contenant 20 g/l en polymère)

En procédant selon la méthode exposée dans les exemples 1 et 2 ci dessus, on a déterminé la solubilité aqueuse de la simvastatine et la nifedipine, tout d'abord en l'absence du polymère POM, puis en présence de polyglutamate de degré de polymérisation 100, greffé à 20 % en vitamine E, à pH 7,0 et concentré à 20 g/l. Les résultats sont rassemblés dans le tableau 3 ci-dessous.

**Tableau 3 : solubilisation de la simvastatine et la nifedipine par une solution aqueuse contenant 20 g/l de polyglutamate de DP = 100, greffé à 20 % en vitamine E, à pH = 7,0, à température ambiante.**

| Actif | Masse molaire M (g/mol) | solubilité (mol/l) | Actif solubilisé pour 20 g/l de polymère (mol/L) | Actif solubilisé en µmol par g de polymère |
|---|---|---|---|---|
| Simvastatine | 419 | 1,19.10⁻⁴ | 7,64.10⁻³ | 382 |
| Nifedipine | 346 | 2,89.10⁻⁵ | 8,67.10⁻⁴ | 43 |

On constate que le polymère polyglutamate de DP 100, greffé à 20 % en vitamine E solubilise de façon spectaculaire ces principes actifs peu solubles. Il faut également noter que des solubilités encore plus élevées peuvent être obtenues avec des concentrations plus fortes en polymère POM.

### Exemple 7

### Préparation de formulations aqueuses de carvédilol base solubilisé par différents polymères POM (formulations contenant 20 g/l en polymère)

Dans cet exemple les polymères POM utilisés sont des polyglutamates de sodium ayant un degré de polymérisation compris entre 25 et 100 et des taux de greffages en vitamine E variables. Sont également testés des polymères dont une partie des glutamates est modifiée par de l'arginine (groupements cationiques) et de l'éthanolamine (groupements neutres).

40 à 50 mg de carvédilol base sont introduits dans un tube Falcon de 15 ml. 5 ml de solution aqueuse de polymère POM concentré à 20 mg/ml et à pH 7,0 sont ajoutés. La préparation est placée dans un bain à ultrasons à température ambiante pendant 90 minutes. Après cette étape, la préparation est centrifugée à 9.000 rpm pendant 30 minutes pour éliminer les éventuels cristaux non dissous. Le surnageant est dans tous les cas parfaitement limpide. Il est dilué 1.000 fois par un tampon phosphate à pH 7,0. La concentration en carvédilol solubilisé est déterminée par spectrométrie UV (à une longueur d'onde de 240 nm et à l'aide de cuve de 1 cm de trajet optique).

**Tableau 4 - solubilisation de carvédilol base par une solution aqueuse contenant 20 g/l de polyglutamate de sodium de DP = 100, greffé avec 2 à 20 % de vitamine E (VE), à pH = 7,0, à température ambiante.**

| % VE sur polymère (%) | Concentration totale d'actif solubilisée pour 20 g/l de polymère (mol/l) | Actif solubilisé en µmol par g de polymère |
|---|---|---|
| 2 | 2,95.10⁻³ | 148 |
| 5 | 6,64.10⁻³ | 332 |
| 10 | 1,53.10⁻² | 763 |
| 20 | 1,53.10⁻² | 763 |

**Tableau 5 - solubilisation de carvédilol base par une solution aqueuse contenant 20 g/l de polyglutamate de sodium de DP variant de 25 à 100, greffé avec 10 % de vitamine E, à pH = 7,0, à température ambiante.**

| Degré de polymérisation du polymère | Concentration totale d'actif solubilisée pour 20 g/l de polymère (mol/l) | Actif solubilisé en µmol par g de polymère |
|---|---|---|
| 25 | 1,48.10⁻² | 738 |
| 50 | 1,35.10⁻² | 677 |
| 100 | 1,53.10⁻² | 763 |

**Tableau 6 - solubilisation de carvédilol base par une solution aqueuse contenant 20 g/l de polyglutamate de sodium de DP = 50, greffé avec 10 % de vitamine E et ayant des greffons cationiques (arginine) et éventuellement neutres (éthanolamine), à pH = 7,0, à température ambiante.**

| % arginine | % éthanolamine | Concentration totale d'actif solubilisée pour 20 g/l de polymère (mol/l) | Actif solubilisé en µmol par g de polymère |
|---|---|---|---|
| 40 | 45 | 1,72.10⁻³ | 86 |
| 60 | 0 | 9,35.10⁻³ | 467 |

La même expérience est réalisée en absence de polymère et l'on trouve que la solubilité du carvédilol base à pH 7 est de 1,2 10⁻⁴ mol/l.

En présence de 20 mg/ml de polymère POM, la solubilité du carvédilol base est donc augmentée d'un facteur 10 à 100 environ selon les polymères. Le tableau 4 permet de voir de plus que pour les polyglutamates de sodium de DP = 100 greffés avec de la vitamine E, le maximum de solubilisation est obtenu pour des polyglutamates greffés avec 10 à 20 % de vitamine E.

### Exemple 8

### Préparation de formulations aqueuses de carvédilol base solubilisé par différents polymères POM (formulations contenant de fortes concentrations en polymère)

Dans cet exemple les polymères POM utilisés sont des polyglutamates de sodium ayant un degré de polymérisation DP = 100 et des taux de greffages en vitamine E variables. La solution de polymère POM est utilisée à une haute concentration de façon à solubiliser le plus possible d'actif. Cependant, de façon à ce que la solution soit facilement manipulable, on maintient une viscosité de la solution < 100 mPa.s (à 20°C), ce qui limite donc la concentration en polymère de façon différente selon les polymères.

50 à 100 mg de carvédilol base sont introduits dans un pilulier. 2 ml de solution aqueuse concentrée de polymère POM à pH 7,0 sont ajoutés. La préparation est placée dans un bain à ultrasons à température ambiante pendant 90 minutes. Dans le cas où il reste des insolubles visibles à l'oeil nu, la solution est replacée pendant 90 minutes supplémentaires dans le bain à ultrasons. La formulation est ensuite laissée à agiter à température ambiante pendant une nuit. Comme dans l'exemple précédent, la formulation est ensuite centrifugée et le surnageant est analysé par HPLC.

**Tableau 7 - solubilisation de carvédilol base par une solution aqueuse concentrée de polyglutamate de sodium de DP = 100, greffé avec 10 à 20 % de vitamine E (VE), à pH = 7,0, à température ambiante.**

| % VE sur polymère (%) | Concentration de la solution de polymère (mg/ml) | Concentration totale d'actif solubilisée (mol/l) | Actif solubilisé en µmol par g de polymère |
|---|---|---|---|
| 10 | 52,8 | 3,37.10⁻² | 638 |
| 15 | 84,6 | 4,01.10⁻² | 474 |
| 20 | 156 | 8,46.10⁻² | 543 |

Le tableau 7 montre que la concentration totale d'actif solubilisé en présence des polymères POM en solution concentrée est bien supérieure à la solubilité du carvédilol base dans l'eau pure au même pH (1,2.10⁻⁴ mol/l). La solubilité est ainsi augmentée d'un facteur compris entre 100 et 1.000.

### Exemple 9

### Préparation de formulations aqueuses de kétoconazole solubilisé par différents polymères POM (formulations contenant 20 g/l en polymère)

Dans cet exemple les polymères POM utilisés sont des polyglutamates de sodium ayant un degré de polymérisation de 100 et des taux de greffages en vitamine E variables. Est également testé un polymère dont une partie des glutamates est modifiée par de l'arginine (groupements cationiques).

2 à 40 mg de poudre de kétoconazole sont introduits dans un tube Falcon de 15 ml. 10 ml de solution aqueuse de polymère POM à pH 7,0 et concentrée à 20 mg/ml, sont ajoutés. La préparation est placée dans un bain à ultrasons à température ambiante pendant 90 minutes. La préparation est ensuite laissée à agiter à température ambiante pendant une nuit. L'aspect de la solution est alors vérifié visuellement pour voir si la poudre de kétoconazole introduite est entièrement solubilisée ou s'il reste des cristaux de kétoconazole résiduels.

**Tableau 8 - solubilisation de kétoconazole par une solution aqueuse contenant 20 g/l de polyglutamate de sodium de DP = 100, greffé avec 2 à 20 % de vitamine E (VE), à pH = 7,0, à température ambiante.**

| % VE sur polymère (%) | Concentration totale d'actif solubilisée pour 20 g/l de polymère (mol/l) | Actif solubilisé en µmol par g de polymère |
|---|---|---|
| 2 | 5,65.10⁻⁴ | 28 |
| 5 | 2,54.10⁻³ | 127 |
| 10 | 7,16.10⁻³ | 358 |
| 15 | 7,16.10⁻³ | 358 |
| 20 | 6,97.10⁻³ | 348 |

Le tableau 8 montre clairement qu'un maximum de solubilisation est obtenu pour les polymères greffés avec 10 à 20 % de vitamine E.

Les mêmes essais sont réalisés avec un polymère cationique.

**Tableau 9 - solubilisation de carvédilol par une solution aqueuse contenant 20 g/l de polyglutamate de sodium de DP = 50, greffé avec 10 % de vitamine E et ayant des greffons cationiques (arginine), à pH = 7,0, à température ambiante.**

| % arginine | Concentration totale d'actif solubilisée pour 20 g/l de polymère (mol/l) | Actif solubilisé en µmol par g de polymère |
|---|---|---|
| 60 | 5,65.10⁻⁴ | 28 |

La même expérience est réalisée en absence de polymère et l'on trouve que la solubilité du kétoconazole à pH 7,0 est d'environ 2.10⁻⁵ mol/l.

En présence de 20 mg/ml de polymère POM, la solubilité du kétoconazole est donc augmentée d'un facteur compris entre 25 et 400 selon les polymères.

### Exemple 10

### Préparation de formulations aqueuses de kétoconazole solubilisé par différents polymères POM (formulations contenant de fortes concentrations en polymère).

Dans cet exemple les polymères POM utilisés sont des polyglutamates de sodium ayant un degré de polymérisation DP = 100 et des taux de greffages en vitamine E variables. La solution de polymère POM est utilisée à une haute concentration de façon à solubiliser le plus possible d'actif. Cependant, de façon à ce que la solution soit facilement manipulable, on maintient une viscosité de la solution < 100 mPa.s (à 20°C) ce qui limite donc la concentration en polymère de façon différente selon les polymères.

10 à 60 mg de poudre de kétoconazole sont introduits dans un pilulier en verre. 2 ml de solution aqueuse concentrée de polymère POM à pH 7,0 sont ajoutés. La préparation est placée dans un bain à ultrasons à température ambiante pendant 90 minutes. Dans le cas où il reste des insolubles visibles à l'oeil nu, la solution est replacée pendant 90 minutes supplémentaires dans le bain à ultrasons. La préparation est ensuite laissée à agiter à température ambiante pendant une nuit. L'aspect de la solution est alors vérifié visuellement pour voir si la poudre de kétoconazole introduite est entièrement solubilisée ou s'il reste des cristaux de kétoconazole résiduels.

**Tableau 10 - solubilisation de kétoconazole par une solution aqueuse concentrée de polyglutamate de sodium de DP = 100, greffé avec 2 à 30 % de vitamine E (VE), à pH = 7,0, à température ambiante.**

| % VE sur polymère (%) | Concentration de la solution de polymère (mg/ml) | Concentration totale d'actif solubilisée (mol/l) | Actif solubilisé en µmol par g de polymère |
|---|---|---|---|
| 10 | 52,8 | 1,60.10⁻² | 303 |
| 15 | 84,6 | 3,11.10⁻² | 367 |
| 20 | 156 | 5,35.10⁻² | 343 |
| 30 | 53,5 | 1,41.10⁻² | 264 |

Le tableau 10 montre que les polymères de DP = 100 contenant entre 15 et 20 % de vitamine E permettent de monter fortement la concentration en polymère tout en maintenant une viscosité acceptable et permettent donc d'obtenir des concentrations d'actif solubilisé importantes (> 3.10⁻² mol/l). Rappelons que la solubilité de l'actif mesurée sans polymère dans des conditions analogues dans l'exemple précédent est de 2.10⁻⁵ mol/l soit plus de 1.000 fois inférieure.

### Exemple 11

### Préparation de formulations aqueuses de cyclosporine-A solubilisé par différents polymères POM (formulations contenant 20 g/l en polymère).

Dans cet exemple les polymères POM utilisés sont des polyglutamates de sodium ayant un degré de polymérisation compris entre 25 et 100 et des taux de greffages en vitamine E variables.

40 à 50 mg de cyclosporine-A sont introduits dans un tube Falcon de 15 ml. 5 ml de solution aqueuse de polymère POM à pH 7,0 et concentrée à 20 mg/ml, sont ajoutés. La préparation est placée dans un bain à ultrasons à température ambiante pendant 90 minutes. La préparation est ensuite laissée à agiter à température ambiante pendant une nuit. La préparation est ensuite centrifugée à 9.000 rpm pendant 30 minutes. Le surnageant est dosé par HPLC afin de déterminer la concentration en cyclosporine-A dans la solution aqueuse.

**Tableau 11 - solubilisation de cyclosporine-A par une solution aqueuse contenant 20 g/l de polyglutamate de sodium de DP = 100, greffé avec 5 à 30 % de vitamine E (VE), à pH = 7,0, à température ambiante.**

| % VE sur polymère (%) | Concentration totale d'actif solubilisée pour 20 g/l de polymère (mol/l) | Actif solubilisé en µmol par g de polymère |
|---|---|---|
| 5 | 4,46.10⁻³ | 223 |
| 10 | 6,24.10⁻³ | 312 |
| 15 | 6,49.10⁻³ | 324 |
| 20 | 5,78.10⁻³ | 289 |
| 30 | 4,03.10⁻³ | 202 |

**Tableau 12 - solubilisation de cyclosporine-A par une solution aqueuse contenant 20 g/l de polyglutamate de sodium de DP entre 25 et 100, greffé avec 10 % de vitamine E, à pH = 7,0, à température ambiante.**

| Degré de polymérisation du polymère | Concentration totale d'actif solubilisée pour 20 g/l de polymère (mol/l) | Actif solubilisé en µmol par g de polymère |
|---|---|---|
| 25 | 5,86.10⁻³ | 293 |
| 50 | 6,16.10⁻³ | 308 |
| 100 | 6,24.10⁻³ | 312 |

La même expérience est réalisée en absence de polymère et l'on trouve que la solubilité de la cyclosporine-A à pH 7 est environ de 2,5.10⁻⁵ mol/l.

En présence de 20 mg/ml de polymère POM la solubilité du kétoconazole est donc augmentée d'un facteur de 200 environ. Le tableau 11 permet de voir de plus que pour les polyglutamates de sodium de DP = 100 greffés avec de la vitamine E, le maximum de solubilisation est obtenu pour des polyglutamates greffés avec 10 à 20 % de vitamine E.

### Exemple 12

### Préparation de formulations aqueuses de cyclosporine-A solubilisé par différents polymères POM (formulations contenant de fortes concentrations en polymère).

Dans cet exemple les polymères POM utilisés sont des polyglutamates de sodium ayant un degré de polymérisation de 100 ou 25 et des taux de greffages en vitamine E variables. La solution de polymère POM est utilisée à une haute concentration de façon à solubiliser le plus possible d'actif. Cependant, de façon à ce que la solution soit facilement manipulable, on maintient une viscosité de la solution < 100 mPa.s (à 20°C) ce qui limite donc la concentration en polymère de façon différente selon les polymères.

60 à 100 mg de cyclosporine-A base sont introduits dans un pilulier. 2 ml de solution aqueuse concentrée de polymère POM à pH 7.0 sont ajoutés. La préparation est placée dans un bain à ultrasons à température ambiante pendant 90 minutes. Dans le cas où il reste des insolubles visibles à l'oeil nu, la solution est replacée pendant 90 minutes supplémentaires dans le bain à ultrasons. La préparation est ensuite laissée à agiter à température ambiante pendant une nuit. La préparation est ensuite centrifugée à 9.000 rpm pendant 30 minutes. Le surnageant est dosé par HPLC afin de déterminer la concentration en cyclosporine-A dans la solution aqueuse.

**Tableau 13 - solubilisation de cyclosporine-A par une solution aqueuse concentrée de polyglutamate de sodium de DP = 100, greffé avec 10 à 20 % de vitamine E (VE), à pH = 7,0, à température ambiante.**

| % VE sur polymère (%) | Concentration de la solution de polymère (mg/ml) | Concentration totale d'actif solubilisée (mol/l) | Actif solubilisé en µmol par g de polymère |
|---|---|---|---|
| 10 | 52,8 | 4,80.10⁻² | 910 |
| 15 | 84,6 | 7,85.10⁻² | 928 |
| 20 | 90,0 | 7,89.10⁻² | 876 |

**Tableau 14 - solubilisation de cyclosporine-A par une solution aqueuse concentrée de polyglutamate de sodium de DP de 25 ou 100, greffé avec 20 % de vitamine E, à pH = 7,0, à température ambiante.**

| Degré de polymérisation | Concentration de la solution de polymère (mg/ml) | Concentration totale d'actif solubilisée (mol/l) | Actif solubilisé en µmol par g de polymère |
|---|---|---|---|
| 100 | 90 | 7,89.10⁻² | 876 |
| 25 | 145 | 9,44.10⁻² | 651 |

Le tableau 13 montre que les polymères de DP = 100 contenant entre 15 et 20 % de vitamine E permettent de monter fortement la concentration en polymère tout en maintenant une viscosité acceptable et permettent donc d'obtenir des concentrations d'actif solubilisé importantes (> 2,6. 10⁻² mol/l). Rappelons que la solubilité de l'actif mesurée sans polymère dans des conditions analogues dans l'exemple précédent est environ de 2,5.10⁻⁵ mol/l, soit plus de 1.000 fois inférieure.

### Exemple 13

### Mesure de la viscosité (mPa/s) sous cisaillement d'une solution aqueuse de polymère POM à la concentration de 50 mg/ml avec un gradient de vitesse de 10 s⁻¹.

Tous les échantillons sont préparés à 50 mg/ml, soit par dilution dans l'eau pure de solutions concentrées dans le cas où la concentration initiale en sortie de synthèse est > 50 mg/ml, soit après concentration sur un évaporateur rotatif dans le cas ou la concentration initiale de la solution est < 50 mg/ml.

La viscosité des solutions aqueuses de polymère est mesurée à 20 °C à l'aide d'un rhéomètre Bohlin de modèle Gemini équipé d'une géométrie de type cône-plan de 4 cm de diamètre et 1° d'angle.

**Tableau 15 - Mesures de viscosité (à 20 °C et pour un gradient de cisaillement de 10 s⁻¹) de solutions à 50 mg/ml de polyglutamates de sodium de différents DP greffés à 5 % en vitamine E (VE).**

| | | Viscosité (mPa.s) |
|---|---|---|
| Degré de polymérisation | 25 | <10 |
| | 50 | <10 |
| | 100 | 25 |
| | 220 | >1.000 |

Ces résultats montrent clairement que pour les taux de greffage en vitamine E de 5 %, un polymère non conforme à l'invention en termes de DP conduit à une viscosité trop élevée.

## Revendications

1. Composition comprenant au moins un actif de solubilité aqueuse inférieure à 1 g/l d'eau pure, ledit actif y étant présent sous une forme associée de manière non covalente à des nanoparticules formées d'au moins un polymère POM de formule (II) suivante ou l'un de ses sels pharmaceutiquement acceptables, 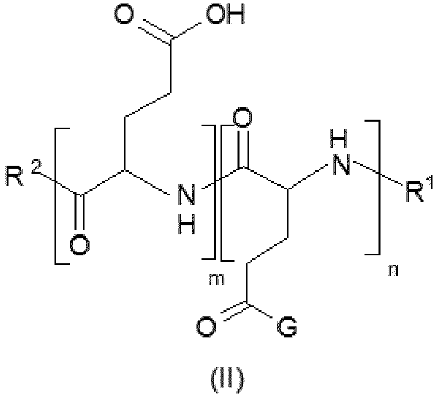 dans laquelle :
- R¹ représente un atome d'hydrogène, un groupe acyle linéaire en C₂ à C₁₀, un groupe acyle ramifié en C₃ à C₁₀, un groupe pyroglutamate ou un groupement hydrophobe G ;
- R² représente un groupe -NHR⁵ ou un résidu acide aminé terminal lié par l'azote et dont le carboxyle est éventuellement substitué par un radical alkylamino -NHR⁵ ou un alcoxy -OR⁶ ;
- R⁵ représente un atome d'hydrogène, un groupe alkyle linéaire en C₁ à C₁₀, un groupe alkyle ramifié en C₃ à C₁₀, ou un groupe benzyle ;
- R⁶ représente un atome d'hydrogène, un groupe alkyle linéaire en C₁ à C₁₀, un groupe alkyle ramifié en C₃ à C₁₀, un groupe benzyle ou un groupement G ;
- G représente un radical tocophéryl ;
- m et n sont des entiers positifs, non nuls ;
- (m+n) varie de 25 à 100 ;
- le taux de greffage molaire des groupements hydrophobes G, (n)/(n+m) varie de 10 à 21 % molaire;
et dans laquelle ledit actif est présent à raison d'au moins 5 µmol/g de POM ;
à l'exclusion d'une composition comprenant du carvédilol base sous une forme associée de manière non covalente à des nanoparticules formées d'un polymère polyglutamate greffé à 20% de vitamine E et présentant un degré de polymérisation d'environ 100, le carvédilol base étant présent à raison d'environ 248 µmol/g de polyglutamate ; et
à l'exclusion d'une composition comprenant du carvédilol base sous une forme associée de manière non covalente à des nanoparticules formées d'un polymère polyglutamate greffé à 10% de vitamine E et présentant un degré de polymérisation d'environ 100, le carvédilol base étant présent à raison d'environ 311 µmol/g de polyglutamate.

2. Composition selon la revendication 1, dans laquelle ledit actif présente une masse inférieure à 2.000 Da.

3. Composition selon la revendication 1 ou 2, dans laquelle ledit actif est non peptidique et de préférence est choisi parmi le paclitaxel, la carvédilol base, la simvastatine, la nifédipine, le kétoconazole et la cyclosporine-A.

4. Composition selon l'une quelconque des revendications précédentes, dans laquelle lesdites nanoparticules sont agglomérées sous formes de microparticules.

5. Composition selon l'une quelconque des revendications 1 à 3, dans laquelle lesdites nanoparticules associées de manière non covalente audit principe actif sont mises en œuvre sous une forme supportée.

6. Composition selon l'une quelconque des revendications 1 à 3, dans laquelle lesdites nanoparticules sont mises en œuvre sous la forme de microparticules, lesdites microparticules possédant un cœur contenant lesdites nanoparticules et au moins une couche d'enrobage conditionnant un profil de libération régulé dudit principe actif en fonction du pH, ladite couche d'enrobage étant formée d'un matériau comprenant au moins un polymère A possédant une valeur de pH de solubilisation comprise dans la plage de pH de 5 à 7 associé à au moins un composé B hydrophobe.

7. Composition selon la revendication précédente, dans laquelle le polymère A est choisi parmi les copolymère(s) d'acide méthacrylique et de méthacrylate de méthyle, le(s) copolymère(s) d'acide méthacrylique et d'acrylate d'éthyle, les dérivés cellulosiques tels que l'acétate phtalate cellulose, l'acétate succinate de cellulose, l'acétate trimellilate de cellulose, le phtalate d'hydroxypropylméthylcellulose, l'acétate succinate d'hydroxypropylméthylcellulose, la gomme shellac, l'acétate phtalate de polyvinyle et leurs mélanges.

8. Composition selon la revendication 6 ou 7, dans laquelle l'enrobage des nanoparticules associées audit principe actif contient de 25 à 90 % en poids, notamment de 30 % à 80 % en poids, en particulier de 35 % à 70 % en poids, voire de 40 à 60 % de polymère(s) A par rapport à son poids total.

9. Composition selon l'une quelconque des revendications 6 à 8, dans laquelle le composé B hydrophobe est sélectionné parmi les produits cristallisés à l'état solide, et ayant une température de fusion T_{fb} ≥ 40 °C, de préférence T_{fb} ≥ 50 °C, et plus préférentiellement encore 40 °C ≤ T_{fb} ≤ 90 °C en particulier choisi parmi les :
- cires végétales ;
- huiles végétales hydrogénées prises seules ou en mélange entre elles ; de préférence choisies dans le groupe comprenant : l'huile de coton hydrogénée, l'huile de soja hydrogénée, l'huile de palme hydrogénée ;
- mono et/ou di et/ou tri esters du glycérol et d'au moins un acide gras, de préférence l'acide béhénique, pris seuls ;
- et leurs mélanges.

10. Composition selon l'une quelconque des revendications 6 à 9, dans laquelle le composé B est un polymère insoluble dans les fluides gastro intestinaux en particulier choisi parmi :
- les dérivés non hydrosolubles de la cellulose et plus particulièrement l'acétate butyrate de cellulose, l'acétate de cellulose,
- les dérivés non hydrosolubles de (co)polymères (méth)acryliques et plus particulièrement les copolymères d'ammonio (méth)acrylate, (co)polymères d'acrylate d'éthyle, de méthacrylate de méthyle et de méthacrylate de triméthylammonio éthyle de type « A » ou de type « B », et les esters d'acides poly(méth)acryliques.

11. Composition selon l'une quelconque des revendications 4 à 10, dans laquelle la taille des microparticules est inférieure à 2.000 µm, en particulier varie de 100 à 1.000 µm, en particulier de 100 à 800 µm et notamment de 100 à 500 µm.

12. Utilisation de nanoparticules d'au moins un polymère POM telles que définies selon l'une quelconque des revendications 1 et 4 à 11, associées de manière non covalente à un actif, ledit actif présentant une solubilité aqueuse inférieure à 1 g/l d'eau pure, en vue d'accroître la solubilisation aqueuse dudit actif, ledit principe actif étant mis en œuvre à raison d'au moins 5 µmol/g de POM.

## Patentansprüche

1. Zusammensetzung, umfassend mindestens einen Wirkstoff mit einer Wasserlöslichkeit von weniger als 1 g/l reines Wasser, wobei der Wirkstoff darin in einer Form vorhanden ist, die nicht kovalent mit Nanopartikeln assoziiert ist, die durch mindestens ein Polymer POM der nachstehenden Formel (II) gebildet werden, oder einem pharmazeutisch verträglichen Salz davon, 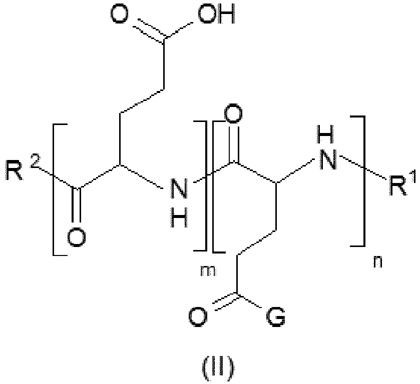 wobei:
- R¹ für ein Wasserstoffatom, eine lineare C₂- bis C₁₀-Acylgruppe, eine verzweigte C₃- bis C₁₀-Acylgruppe, eine Pyroglutamatgruppe oder eine hydrophobe Gruppe G steht;
- R² für eine Gruppe -NHR⁵ oder einen terminalen Aminosäurerest steht, der an den Stickstoff gebunden ist und dessen Carboxyl gegebenenfalls durch einen Alkylaminorest -NHR⁵ oder einen Alkoxyrest -OR⁶ substituiert ist;
- R⁵ für ein Wasserstoffatom, eine lineare C₁- bis C₁₀-Alkylgruppe, eine verzweigte C₃- bis C₁₀-Alkylgruppe oder eine Benzylgruppe steht;
- R⁶ für ein Wasserstoffatom, eine lineare C₁- bis C₁₀-Alkylgruppe, eine verzweigte C₃- bis C₁₀-Alkylgruppe oder eine Benzylgruppe oder eine Gruppe G steht;
- G für einen Tocopherylrest steht;
- m und n positive ganze Zahlen ungleich null sind;
- (m+n) zwischen 25 bis 100 liegt;
- der Pfropfgrad in Mol der hydrophoben Gruppen G (n)/(n+m) zwischen 10 und 21 Mol-% liegt;
und der Wirkstoff in einer Menge von mindestens 5 µmol/g POM vorhanden ist;
mit Ausnahme einer Zusammensetzung, umfassend die Carvedilol-Basis in einer nicht kovalent mit Nanopartikeln assoziierten Form, die aus einem Polyglutamatpolymer gebildet sind, das mit 20 % Vitamin E gepfropft ist und einen Polymerisationsgrad von etwa 100 aufweist, wobei die Carvedilol-Basis in einer Menge von etwa 248 µmol/g Polyglutamat vorhanden ist; und
mit Ausnahme einer Zusammensetzung, umfassend die Carvedilol-Basis in einer nicht kovalent mit Nanopartikeln assoziierten Form, die aus einem Polyglutamatpolymer gebildet sind, das mit 10 % Vitamin E gepfropft ist und einen Polymerisationsgrad von etwa 100 aufweist, wobei die Carvedilol-Basis in einer Menge von etwa 311 µmol/g Polyglutamat vorhanden ist.

2. Zusammensetzung nach Anspruch 1, wobei der Wirkstoff eine Masse von weniger als 2.000 Da aufweist.

3. Zusammensetzung nach Anspruch 1 oder 2, wobei der Wirkstoff nicht peptidartig ist und bevorzugt ausgewählt ist aus Paclitaxel, Carvedilol-Basis, Simvastatin, Nifedipin, Ketoconazol und Cyclosporin-A.

4. Zusammensetzung nach einem der vorhergehenden Ansprüche, wobei die Nanopartikel in Form von Mikropartikeln agglomeriert sind.

5. Zusammensetzung nach einem der Ansprüche 1 bis 3, wobei die mit dem Wirkstoff nicht kovalent assoziierten Nanopartikel in einer geträgerten Form eingesetzt werden.

6. Zusammensetzung nach einem der Ansprüche 1 bis 3, wobei die Nanopartikel in Form von Mikropartikeln eingesetzt werden, wobei die Mikropartikel einen Kern, der die Nanopartikel enthält, und mindestens eine Überzugsschicht aufweisen, die ein geregeltes Freisetzungsprofil des Wirkstoffs in Abhängigkeit vom pH-Wert konditioniert, wobei die Überzugsschicht aus einem Material gebildet ist, das mindestens ein Polymer A umfasst, das einen Solubilisierungs-pH-Wert im pH-Bereich von 5 bis 7 aufweist, assoziiert mit mindestens einer hydrophoben Verbindung B.

7. Zusammensetzung nach dem vorhergehenden Anspruch, wobei das Polymer A ausgewählt ist aus Copolymer(en) von Methacrylsäure und Methylmethacrylat, Copolymer(en) von Methacrylsäure und Ethylacrylat, Cellulosederivaten, wie etwa Celluloseacetatphthalat, Celluloseacetatsuccinat, Celluloseacetattrimellilat, Hydroxypropylmethylcellulosephthalat, Hydroxypropylmethylcelluloseacetatsuccinat, Schellackgummi, Polyvinylacetatphthalat und Mischungen davon.

8. Zusammensetzung nach Anspruch 6 oder 7, wobei der Überzug der mit dem Wirkstoff assoziierten Nanopartikel 25 bis 90 Gew.-%, bevorzugt 30 Gew.-% bis 80 Gew.-%, besonders bevorzugt 35 Gew.-% bis 70 Gew.-% oder sogar 40 bis 60 Gew.-% Polymer(e) A, bezogen auf das Gesamtgewicht davon, enthält.

9. Zusammensetzung nach einem der Ansprüche 6 bis 8, wobei die hydrophobe Verbindung B ausgewählt ist aus Produkten, die im festen Zustand kristallin sind und eine Schmelztemperatur T_{fb} ≥ 40 °C, bevorzugt T_{fb} ≥ 50 °C und noch mehr bevorzugt 40 °C ≤ T_{fb} ≤ 90 °C aufweisen, ausgewählt aus:
- pflanzlichen Wachsen;
- hydrierten pflanzlichen Ölen, allein oder als Mischung; vorzugsweise ausgewählt aus der Gruppe umfassend: hydriertes Baumwollsamenöl, hydriertes Sojaöl, hydriertes Palmöl;
- Mono- und/oder Di- und/oder Triestern von Glycerol und mindestens einer Fettsäure, bevorzugt Behensäure, allein;
- und Mischungen davon.

10. Zusammensetzung nach einem der Ansprüche 6 bis 9, wobei die Verbindung B ein Polymer ist, das in den Magen-Darm-Flüssigkeiten unlöslich ist, insbesondere ausgewählt aus:
- wasserunlöslichen Cellulosederivaten und insbesondere Celluloseaceatbutyrat, Celluloseacetat,
- wasserunlöslichen Derivaten von (Meth)acryl-(Co)polymeren und insbesondere Ammonium(meth)acrylat-Copolymeren, (Co)polymeren von Ethylacrylat, Methylmethacrylat und TrimethylammoniumEthylmethacrylat vom Typ "A" oder vom Typ "B", und Poly(meth)acrylsäureestern.

11. Zusammensetzung nach einem der Ansprüche 4 bis 10, wobei die Größe der Mikropartikel weniger als 2.000 µm, bevorzugt zwischen 100 und 1.000 µm, bevorzugt zwischen 100 und 800 µm und insbesondere zwischen 100 und 500 µm beträgt.

12. Verwendung von Nanopartikeln mindestens eines Polymers POM, definiert gemäß einem der Ansprüche 1 und 4 bis 11, die nicht kovalent mit einem Wirkstoff assoziiert sind, wobei der Wirkstoff eine Wasserlöslichkeit von weniger als 1 g/l reines Wasser aufweist, um die Wasserlöslichkeit des Wirkstoffs zu erhöhen, wobei der Wirkstoff in einer Menge von mindestens 5 µmol/g POM eingesetzt wird.

## Claims

1. Composition comprising at least one active ingredient with an aqueous solubility of less than 1 g/l of pure water, said active ingredient being present therein in a form noncovalently associated with nanoparticles formed by at least one POM polymer of formula (II) below, or a pharmaceutically acceptable salt thereof: 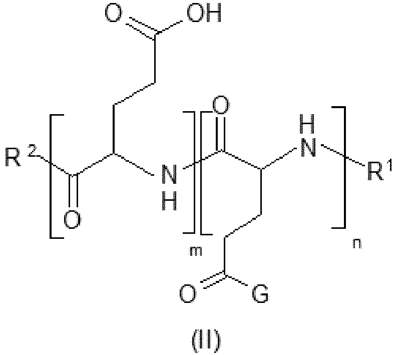 in which:
- R¹ represents a hydrogen atom, a linear C₂ to C₁₀ acyl group, a branched C₃ to C₁₀ acyl group, a pyroglutamate group or a hydrophobic group G;
- R² represents a -NHR⁵ group or a terminal amino acid residue linked by the nitrogen and the carboxyl of which is optionally substituted with an alkylamino radical -NHR⁵ or an alkoxy -OR⁶;
- R⁵ represents a hydrogen atom, a linear C₁ to C₁₀ alkyl group, a branched C₃ to C₁₀ alkyl group or a benzyl group;
- R⁶ represents a hydrogen atom, a linear C₁ to C₁₀ alkyl group, a branched C₃ to C₁₀ alkyl group, a benzyl group or a group G;
- G represents a tocopheryl radical;
- m and n are positive, non-zero integers;
- (m+n) ranges from 25 to 100;
- the molar degree of grafting of the hydrophobic groups G, (n)/(n+m) ranges from 10 to 21%.
and in which said active ingredient is present in a proportion of at least 5 µmol/g of POM,
with exclusion of a composition comprising carvedilol base in a form noncovalently associated with nanoparticles formed by a polyglutamate polymer which is 20%-grafted with vitamin E and has a degree of polymerization of approximately 100, said carvedilol base being present in a proportion of approximately 248 µmol/g of polyglutamate; and
with exclusion of a composition comprising carvedilol base in form noncovalently associated with nanoparticles formed by a polyglutamate polymer which is 10%-grafted with vitamin E and has a degree of polymerization of approximately 100, said carvedilol base being present in a proportion of approximately 311 µmol/g of polyglutamate .

2. Composition according to claim 1, in which said active ingredient has a mass of less than 2,000 Da.

3. Composition according to claim 1 or 2, in which said active ingredient is a nonpeptide active ingredient and preferably is chosen from paclitaxel, carvedilol base, simvastatin, nifedipine, ketoconazole, and cyclosporin A.

4. Composition according to any one of the previous claims, in which said nanoparticles are agglomerated in the form of microparticles.

5. Composition according to any one of Claims 1 to 3, in which said nanoparticles noncovalently associated with said active ingredient are used in a supported form.

6. Composition according to any one of Claims 1 to 3, in which said nanoparticles are used in the form of microparticles, said microparticles having a core containing said nanoparticles and at least one coating layer conditioning a release profile, for said active ingredient, which is regulated as a function of pH, said coating layer being formed by a material comprising at least one polymer A having a solubilization pH value within the pH range of 5 to 7, associated with at least one hydrophobic compound B.

7. Composition according to the preceding claim, in which the polymer A is chosen from copolymer(s) of methacrylic acid and methyl methacrylate, copolymer(s) of methacrylic acid and ethyl acrylate, cellulosic derivatives such as cellulose acetate phthalate, cellulose acetate succinate, cellulose acetate trimellitate, hydroxypropylmethylcellulose phthalate, hydroxypropylmethylcellulose acetate succinate, shellac gum, polyvinyl acetate phthalate, and mixtures thereof.

8. Composition according to Claim 6 or 7, in which the coating of the nanoparticles associated with said active ingredient contains from 25% to 90% by weight, especially from 30% to 80% by weight, in particular from 35% to 70% by weight, or even from 40% to 60% of polymer(s) A relative to its total weight.

9. Composition according to any one of Claims 6 to 8, in which the hydrophobic compound B is selected from products that are crystalline in the solid state and that have a melting temperature T_{mb} ≥ 40°C, preferably T_{mb} ≥ 50°C, and even more preferably 40°C ≤ T_{mb} ≤ 90°C, in particular chosen from:
- vegetable waxes;
- hydrogenated vegetable oils taken alone or as a mixture with one another; preferably chosen from the group comprising: hydrogenated cotton oil, hydrogenated soya oil, hydrogenated palm oil;
- mono- and/or di- and/or triesters of glycerol and of at least one fatty acid, preferably behenic acid, taken alone;
- and mixtures thereof.

10. Composition according to any one of Claims 6 to 9, in which the compound B is a polymer that is insoluble in the gastrointestinal fluids, in particular chosen from:
- water-insoluble derivatives of cellulose, and more particularly cellulose acetate butyrate and cellulose acetate,
- water-insoluble derivatives of (meth)acrylic (co)polymers, and more particularly ammonio (meth)acrylate copolymers, (co)polymers of ethyl acrylate, methyl methacrylate and trimethylammonioethyl methacrylate, type "A" or type "B", and poly(meth)acrylic acid esters.

11. Composition according to any one of Claims 4 to 10, in which the size of the microparticles is less than 2,000 µm, in particular ranges from 100 to 1,000 µm, in particular from 100 to 800 µm, and especially from 100 to 500 µm.

12. Use of nanoparticles of at least one POM polymer as defined in any one of Claims 1 and 4 to 11, noncovalently associated with an active ingredient, said active ingredient having an aqueous solubility of less than 1 g/l of pure water, with a view to increasing the aqueous solubilization of said active ingredient, said active ingredient being used in a proportion of at least 5 µmol/g of POM.
